# EUROPEAN PATENT SPECIFICATION

(11) **EP 0 587 565 B1**
(45) Date of publication and mention of the grant of the patent: **19.08.1998**
(21) Application number: 92904476.6
(22) Date of filing: 06.06.1991
(51) Int. Cl.: A61F 2/16, A61L 27/00

(54) **INTRAOCULAR LENS WITH HAPTIC ANCHOR PLATE**
INTRAOKULÄRE LINSE MIT EINER HAPTISCHEN VERANKERUNGSPLATTE
LENTILLE INTRAOCULAIRE POURVUE D'UNE PLAQUETTE D'ANCRAGE HAPTIQUE

(43) Date of publication of application: 23.03.1994
(73) Proprietor: MEDEVEC LICENSING B.V., 1075 AD Amsterdam (NL)
(72) Inventor: Cumming, J. Stuart, Anaheim, California 92801 (US)
(74) Representative: Molyneaux, Martyn William
(86) International application number: US9104001
(87) International publication number: WO9221304

(56) References cited:
- EP-A- 0 175 972
- DE-A- 2 556 665
- GB-A- 2 124 500
- US-A- 4 056 855
- US-A- 4 242 762
- US-A- 4 244 060
- US-A- 4 342 123
- US-A- 4 673 406
- US-A- 4 726 367
- US-A- 4 826 832
- US-A- 5 047 051

## Description

### BACKGROUND OF THE INVENTION

Surgical removal of the opaque lens from the eyes of cataract patients is one of the most common surgical procedures. In the past, contact lenses or spectacles were usually prescribed for the patient to provide at least limited vision following the operation. However, there were many drawbacks to the use of contact lenses and spectacles for such purposes. The present-day practice involves the implantation of an artificial intraocular lens to replace the removed opaque human lens as a preferred procedure to restore the patient's sight. The first intraocular lens was inserted in 1949 by Harold Ridley in England.

The eye is divided by the iris into an anterior chamber in front of the iris and a posterior chamber behind the iris and in front of the human lens. The known intraocular lens can be placed either in the anterior chamber or the posterior chamber. Placement in the posterior chamber has been preferred because the lens can simply be positioned by the use of centering haptic loops or the like extending from the lens body. Such an intraocular lens is described, for example, in US-A-4,634,441 (Clayman et al.). In this reference, which is believed to form the closest prior art, an intraocular lens is disclosed for positioning in the posterior chamber of the eye of a recipient. The intraocular lens has a generally disc-shaped optic portion which is circumferentially surrounded by a carrier. The carrier, therefore, forms a haptic anchor plate which extends radially outwardly from the optic portion. A pair of haptic members are attached to the carrier on diametrically opposite sides thereof and the anchor plate, the optic portion and the haptic members are generally co-planar with one another. This reference further discloses protrusions on the rear surface of the carrier to space the intraocular lens from the posterior capsule. The present invention is concerned with an intraocular lens which is intended for location in the capsular bag of the eye of a recipient.

Until recent years intraocular lenses have been constructed of hard material, such as glass or plastic. Typically, the lens body of the prior art intraocular lens is formed of polymethylmethacrylate (PMMA) and the haptics have been formed of polypropylene (PROLENE). However, PMMA is a hard material and the lenses made from this material usually have a diameter of between 6 and 7mm. This requires an incision of 7 - 8mm in order to insert the lens into the eye. Accordingly, proposals have been made in the past to form the intraocular lens of a soft flexible material such as silicone or hydrophilic polymer which can be folded.

Moreover, those skilled in the art of cataract extraction have long recognized the need to develop a foldable or compressible lens that will pass through a small incision of 4 mm or less. The advantages of a small incision include reduced complications, more rapid visual and physical rehabilitation, and reduced costs. A method for implanting a deformable intraocular lens into the eye is described, for example, in U.S. Patent 4,573,998. Deformable intraocular lenses made primarily of HEMA (hydroxyethylmethylmethacrylate) and silicone have been developed that will pass through incisions of 4 mm or less.

However, difficulties have been encountered in the design of soft deformable intraocular lenses, and such problems have consisted mainly in staking the haptic onto the soft optical zone of the intraocular lens. The PROLENE haptic, when anchored onto the optical zone, necessitates thickening the edge of the optical zone in order to accommodate the staked end of haptic. This reduces the useful optical zone of the lens down to 4.5 mm when it should be 6 mm. In addition, it is proven difficult to attach the haptic to the optical zone of the soft deformable intraocular lens, since it is essential for the haptic to have a constant angular relationship with the optical zone. For that reason, the rejection rate in manufacturing present-day soft intraocular lenses is high and, therefore, the cost of acceptable soft deformable intraocular lenses is also high.

It is well known that some of the prior art intraocular lenses are misplaced during insertion so that one or both of the haptic loops are inadvertently inserted into the sulcus. The sulcus is longer than the capsular bag into which the lens was intended to be placed, and thus the prior art intraocular lens must have haptic loops long enough to fix the intraocular lens into the sulcus should it be misplaced. Otherwise, the misplaced lens could be decentered within the eye.

It is known to attach the haptic loops to the optical region of an intraocular lens. Such a construction is disclosed, for example, in US Patents, 4,834,751 and 4,790,846. However, the present invention provides a soft deformable intraocular lens having a relatively rigid haptic anchor plate attached to the opposite ends of the optical region of the lens. The haptic anchor plate is designed so that there is no loss of the optical zone. The anchor plate provides a base into which relatively short looped haptics formed, for example, of PROLENE or polyamide, can be staked, allowing for a large staking area of adequate thickness, without affecting the dimensions of the optical zone itself. Moreover, the haptic anchor plate makes decentration of the intraocular lens impossible when the lens is placed in the capsular bag, because the anchor plates are rigid enough to resist deformation when capsule fibrosis occurs, and because the length of the lens is at least as long as the diameter of the capsular bag.

German publication DE-A-25 56 665 (Titmus), considered as the closest prior art to the present invention, shows an intraocular lens with haptic loops but is devoid of any arcuate resilient haptic members attached to an anchor plate which can be compressed down adjacent to the end edges of the haptic anchor plate when the lens is positioned in the capsular bag of the eye of the recipient, and which spring outwardly from the ends of the haptic anchor plate when the lens is positioned in the sulcus of the eye of the recipient for positioning the intraocular lens in the sulcus. The haptic elements of DE-A-25 56 665 are only fixed positioning members for locating the lens in the center of the capsular bag. Because the ends of the haptic loops are attached to an anchor plate, there is no possibility for the haptic elements to collapse or to be compressed down adjacent to the end edges of the haptic anchor plate.

U.S. Patent No. 4,056,855 (C. Kelman) shows an intraocular lens having a supporting wire frame, but lacks any element which even remotely suggests the provision of grooves formed in the ends of a haptic anchor plate for receiving compressed arcuate resilient haptic members as is preferred in the present invention, primarily because this reference does not suggest the use of any arcuate haptic members that are compressible, aside from the fact that the ends of the anchor plate (pair of curved tongue portions 30 and 32) are not grooved and extend as a cantilever free body which has no function whatsoever for receiving any other element of the device.

U.S. Patent No. 4,244,060 discloses an intraocular lens which includes a lens body and a plurality of lens-centering filaments extending radially outwardly in a common plane from spaced-rim portions of the lens body. The lens is particularly adapted for implantation in the posterior chamber of the eye. When so positioned, the ends of the filaments are inserted into the cleft of the capsular bag, and the resilience of the filaments centers the lens behind the pupil. The filaments of this reference are essentially configured radially within the capsular bag not permitting tangential fixation by fibrosis.

European publication A-0, 175,972 (Kalb) describes a hard non-flexible lens. Centration is achieved by means of long resilient loops not by plates which are sized to be the length of the capsular bag. The plate into which the loops are staked completely surround the optic and are relatively much shorter in diameter than the capsular bag.

### SUMMARY OF THE INVENTION

In accordance with the invention, there is provided an intraocular lens for an eye of a recipient comprising:
a generally disc-shaped optic portion;
a haptic anchor plate extending radially outwardly from said optic portion in opposite directions therefrom and attached thereto;
a pair of haptic members attached to said anchor plate at opposite ends thereof, said haptic anchor plate, said optic portion and said haptic members being co-planar with one another,
said haptic anchor plate having a length adapted to correspond to the transverse dimension of the capsular bag of the eye of the recipient;
said haptic anchor plate having a width substantially equal to that of the diameter of said optic portion; and
said haptic members being arcuate resilient members at least one of which can be compressed down toward an end edge of the haptic anchor plate during positioning of the intraocular lens in the capsular bag and which at least one member is moveable outward of the haptic anchor plate for alternative positioning of the intraocular lens in the sulcus, whereby said at least one member is for positioning the intraocular lens centrally in the eye of the recipient.

Preferably, the intraocular lens of the invention employs a flexible material, a semi-rigid haptic anchor plate and relatively short haptic loops attached to the anchor plate provide a construction which enables the intraocular lens to be compressed for insertion through a small incision without damage to the haptic loops. This is a preferred feature, because it has been found that attempts to compress soft intraocular lenses having relatively long haptic loops attached to the optical region is difficult and frequently causes damage to the lens. In addition, the possibility of decentration after insertion of the lens is reduced because the semi-rigid haptic anchor plate resists folding or compression by the fibrosis of the human capsular bag, unlike the long PROLENE haptics of conventional intraocular lenses.

In one embodiment of the invention to be described, grooves are formed at the ends of the rigid haptic anchor plate which receive the haptic loops when the intraocular lens is correctly placed in the capsular bag. During such placement, the ends of the haptic anchor plate containing the haptic loops engage the margins of the capsular bag holding the intraocular lens correctly centered and in place. However, if the intraocular lens is misplaced into the sulcus, the haptic loops will extend beyond the ends of the anchor plate and press against the sulcus and stabilize the intraocular lens within the eye. Accordingly, complications are prevented should the intraocular lens be misplaced within the eye, which studies have revealed to happen approximately 50% of the time.

The intraocular lens of the invention can be folded and compressed through a 3-4 mm phacoemulsification incision, or it can be used in the routine planned extracapsular cataract extraction with a large 15 mm incision. Decentration of the intraocular lens of the invention is virtually eliminated when the lens is placed in the capsular bag, as mentioned above, due to the haptic anchor plate, this plate being used not only to provide a base for affixing the haptic loops to the lens, but also for lens fixation in the capsular bag, with the flexible haptic loops being used when the lens is intentionally or accidentally placed in the sulcus.

In the lens of the invention, the polyamide or PROLENE haptic loops are staked into two thickened edges of the haptic anchor plate. As mentioned above, this allows for a large staking area without any reduction in the optical zone of the intraocular lens. This is a distinct improvement over conventional intraocular lenses which have the haptic loops staked directly into the optic zone, thus reducing the optic zone of the lens between the stakes. The staking is done on the edge of the haptic anchor plate to facilitate folding of the intraocular lens when it is used in small incision surgery.

As will be described, grooves at the end of the haptic anchor plate allows the haptic loops to be compressed into the haptic anchor plate during the compression of the intraocular lens for insertion through a small incision. This protects the haptic loops from damage during compression and insertion of the intraocular lens through a small incision.

Also, the overall length of the lens and anchor plate is preferably made equal to the transverse dimension of the capsular bag, and the anchor plate is made sufficiently rigid, so that the posterior capsule is pulled tight against the posterior surface of the lens to prevent opacification of the posterior capsule.

The prior art resilient loop haptic lenses have a tendency to decenter because the haptic loops have very little resistance to forces of fibrosis of the capsular bag during healing. The intraocular lens of the present invention with the relatively rigid integral haptic anchor plate, which has a length corresponding to the length of the lens bag into which it is to be fitted, effectively eliminates any tendency for the intraocular lens to decenter.

The haptic anchor plate is preferably made of silicone, and has sufficient rigidity to resist the forces of fibrosis. Accordingly, as mentioned above, there is no tendency for the intraocular lens of the present invention to decenter. The haptic loops preferably extend to 13 mm so that if the intraocular lens is inadvertently placed in the sulcus, it will remain centered, as explained above. The intraocular lens of the invention also resists deformation when it is "shrink wrapped" by the capsular bag into which it has been inserted. This latter action causes the posterior elastic capsule to be pulled tightly against the posterior surface of the intraocular lens and greatly reduced opacification of the posterior capsule.

### BRIEF DESCRIPTION OF THE DRAWINGS

FIGURE 1 is a top plan view of an intraocular lens and haptic anchor plate constructed in accordance with one embodiment of the invention.

FIGURE 2 is a sectional view taken substantially along the line 2-2 of FIGURE 1.

FIGURES 3-5 are top plan views of further embodiments.

### DETAILED DESCRIPTION OF THE ILLUSTRATED EMBODIMENT

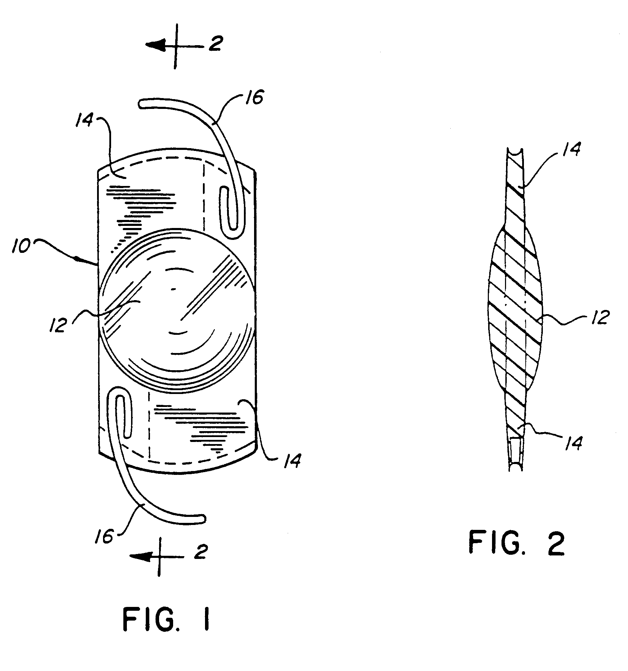

The intraocular lens of FIGURES 1 and 2 is designated generally as 10 which may be a single or multi-focal optical system. The lens includes a central disc-shaped optic portion 12 and an essentially rectangular haptic anchor plate 14 attached to the optic portion.

As best shown in FIGURE 1, the haptic anchor plate 14 has a width essentially equal to the diameter of the optic portion 12, and a length greater than the diameter of the optic portion. The haptic anchor plate 14 extends radially outwardly from the optic portion 12 at diametrically opposite ends of the optic portion, as shown in FIGURE 1. The haptic anchor plate is sufficiently rigid to prevent deformation during fibrosis, as explained above. As also explained, the length of the haptic anchor plate corresponds to that of the capsular bag in which the lens is intended to be placed. These two factors prevent decentration of the intraocular lens when placed in the capsular bag.

In accordance with the invention, the intraocular lens 10 of FIGURE 1 includes a pair of haptic loops 16 which are staked to the extended end portions of the anchor plate 14. The haptic loops 16 are shorter than those usually employed in prior art intraocular lenses, this being possible because of the length of the haptic anchor plate 14. The haptic loops are staked to a thickened part of the haptic anchor plate 14 to one side, as shown in FIGURE 1, to facilitate folding of the intraocular lens.

As shown in FIGURES 1 and 2, grooves are formed in the ends of the haptic anchor plate 14 which serve to receive the haptic loops 16 when the haptic loops are compressed down into the grooves. The haptic loops 16 are completely compressed into the grooves when the intraocular lens is placed in the capsular bag. For such a placement, the lens is held in position by the haptic anchor plate 14, and rigidity of the haptic anchor plate maintains the lens centered in the capsular bag.

However, should the lens be placed in the sulcus, the loops 16 spring out of the grooves, and engage the sides of the sulcus so that the lens is firmly held in the sulcus, should that placement occur.

Accordingly, the construction of the intraocular lens of the invention, as shown in FIGURES 1 and 2, assures that there will be a predetermined orientation of the intraocular lens in the eye, and also assure that the intraocular lens will not be subject to angular or linear movement within the eye, regardless of whether the lens is properly placed in the capsular bag, or it is placed in the sulcus. In addition, the short haptic loops are staked to the haptic anchor plate 14 at positions such that the intraocular lens 10 may be compressed for insertion through a small incision without damage to the haptic members. Also, and as described above, decentration of the intraocular lens within the eye is minimized because the semi-rigid haptic anchor plate 14 resists folding and compression, unlike the relatively long haptic loops of the conventional intraocular lenses.

The optic portion 12 of the intraocular lens of the invention may be formed of a soft foldable transparent plastic such as hydroxyethylmethylmethacrylate. The haptic anchor plate 14 may be formed of any appropriate relatively rigid material. The haptic loops 16 may be formed, for example, of polyamide or polypropylene (PROLENE), or other appropriate resilient material.

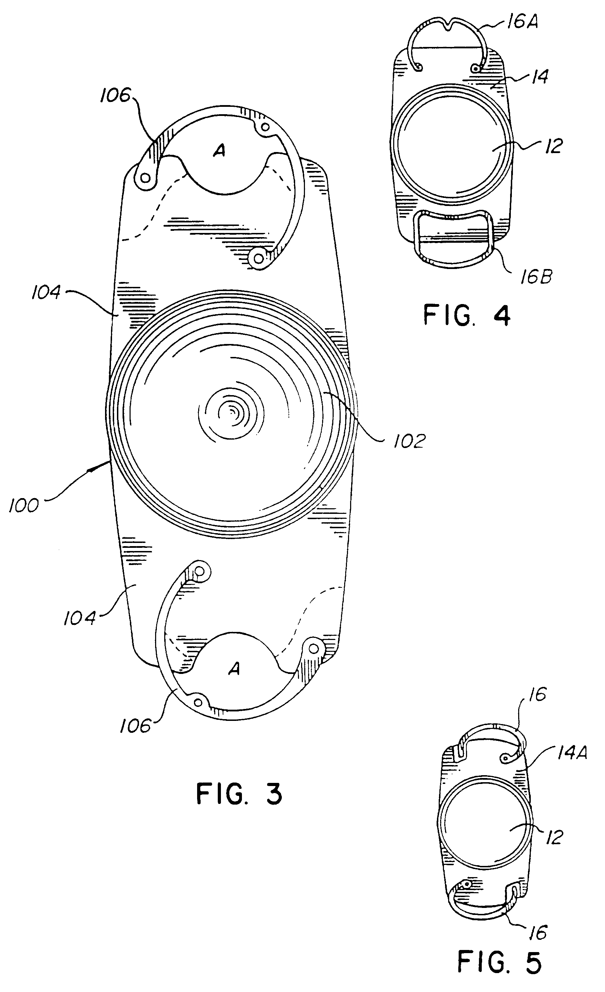

The intraocular lens of FIGURE 3 is designated 100. It includes a central disc-shaped optic portion 102 and an anchor plate 104. A pair of haptic loops 106 are staked to the extended end portions of the anchor plate 104. One or both ends of each haptic loop 106 may be staked to the anchor plate 104. The haptic loops 106 and the haptic anchor plate 104 are configured, as shown, so that spaces "A" are left between the loops and the anchor plate.

The spaces permit the anterior and posterior capsules to fuse together by fibrosis over the loops after the intraocular lens has been inserted into the bag and the loops have been compressed. This fusion firmly fixes the intraocular lens in the bag and prevents its dislocation should it become necessary to open the posterior capsule with a Y.A.G. Laser at a later date.

FIGURE 4 shows an embodiment in which both ends of the haptic loops 16A, and 16B are anchored to the anchor plate 14. Loop 16A is notched for insertion purposes.

FIGURE 5 shows an embodiment in which each end of the haptic anchor plate 14A is notched to receive the end of the corresponding haptic 16. The haptics are pushed all the way into the notches when the intraocular lens is placed in the bag, and partially into the notches when the lens is placed in the sulcus.

The invention provides, therefore, an improved intraocular lens/anchor plate combination which enables haptic members to be attached to the lens without any reduction in the optic region, and which exhibits other distinct advantages set forth above.

It will be appreciated that while particular embodiments of the invention have been shown and described, modifications may be made. It is intended in the claims to cover all modifications which come within the scope of the invention.

## Claims

1. An intraocular lens (10, 100) for an eye of a recipient comprising:
a generally disc-shaped optic portion (12, 102);
a haptic anchor plate (14, 14A, 104) extending radially outwardly from said optic portion (12, 102) in opposite directions therefrom and attached thereto;
a pair of haptic members (16, 16A, 16B, 106) attached to said anchor plate (14, 14A, 104) at opposite ends thereof, said haptic anchor plate, said optic portion and said haptic members being co-planar with one another,
said haptic anchor plate having a length adapted to correspond to the transverse dimension of the capsular bag of the eye of the recipient;
said haptic anchor plate (14, 14A, 104) having a width substantially equal to that of the diameter of said optic portion (12, 102); and
said haptic members (16, 16A, 16B, 106) being arcuate resilient members (16, 16A, 16B, 106) at least one of which can be compressed down toward an end edge of the haptic anchor plate (14, 14A, 104) during positioning of the intraocular lens (10, 100) in the capsular bag and which at least one member is moveable outward of the haptic anchor plate (14, 14A, 104) for alternative positioning of the intraocular lens in the sulcus, whereby said at least one member is for positioning the intraocular lens (10, 100) centrally in the eye of the recipient.

2. The intraocular lens (10, 100) defined in claim 1, wherein said haptic loop members (16, 16A, 16B, 106) are formed of polypropylene.

3. The intraocular lens (10, 100) defined in claim 1, wherein at least one of said optic portion (12, 102) and said anchor plate (14, 14A, 104) is formed of hydroxyethylmethylmethacrylate.

4. The intraocular lens (10, 100) defined in claim 1, wherein at least one of said optic portion (12, 102), said anchor plate (14, 14A, 104) and said haptic members (16, 16A, 16B, 106) are formed of a relatively hard rigid plastic material.

5. The intraocular lens (10, 100) defined in claim 1, wherein said haptic members (16, 16A, 16B, 106) are formed of polyamide.

6. The intraocular lens (10, 100) defined in claim 1, wherein at least one of said optic portion (12, 102), said haptic members (16, 16A, 16B, 106) and said anchor plate (14, 14A, 104) are formed of silicone.

7. The intraocular lens (10, 100) defined in claim 1, wherein at least one of said optic portion (12, 102), said anchor plate (14, 14A, 104) and said haptic members (16, 16A, 16B, 106) are formed of a semi-rigid plastic material.

8. The intraocular lens (10, 100) defined in any preceding claim, wherein the edges of said haptic anchor plate (14, 14A, 104) at the respective ends thereof have grooves formed therein to receive the arcuate haptic members (16, 16A, 16B, 106) when the haptic members (16, 16A, 16B, 106) are compressed down into the grooves by the capcular bag.

9. The intraocular lens (10, 100) defined in claim 1, wherein said optic portion (12, 102) is formed of foldable plastic material.

10. The intraocular lens (10, 100) defined in claim 1, wherein at least one of said optic portion (12, 102), said anchor plate (14, 14A, 104) and said haptic members (16, 16A, 16B, 106) are formed of a resilient plastic material.

11. The intraocular lens (10, 100) defined in any preceding claim, configured so that a space is provided between each of the haptic members (16, 16A, 16B, 106) and the anchor plate (14, 14A, 104) when the intraocular lens (10, 100) is inserted into the capsular bag to permit the anterior and posterior capsules of the eye to fuse together through said space by fibrosis and firmly fix the intraocular lens (10, 100) in the eye.

12. The intraocular lens (10, 100) defined in any preceding claim, wherein each of said haptic members (16, 16A, 16B, 106) is staked at one end only to said anchor plate (14, 14A, 104).

13. The intraocular lens (10, 100) defined in claim 1, wherein each of said arcuate resilient haptic members (16, 16A, 16B, 106) is compressible and attached at both ends to said anchor plate (14, 14A, 104).

14. The intraocular lens (10, 100) defined in claim 12, wherein each end of said anchor plate (14, 14A, 104) is notched to receive the free end of the corresponding haptic members (16, 16A, 16B, 106).

15. The intraocular lens (10, 100) defined in any preceding claim, wherein the intraocular lens (10, 100) is compressible for insertion through a 3-4mm phacoemulsification incision in the eye of the recipient into which the intraocular lens (10, 100) is to be inserted.

## Patentansprüche

1. Intraokulare Linse (10, 100) für ein Auge eines Empfänger, umfassend:
einen im allgemeinen scheibenförmigen optischen Teil (12, 102);
eine haptische Ankerplatte (14, 14A, 104), die sich von dem optischen Teil (12, 102) radial nach außen in entgegengesetzten Richtungen davon erstreckt;
ein Paar haptischer Elemente (16, 16A, 16B, 106), die an der Ankerplatte (14, 14A, 104) an den gegenüberliegenden Seiten davon angebracht sind, wobei die haptische Ankerplatte, der optische Teil und die haptischen Elemente zueinander koplanar sind;
wobei die haptische Ankerplatte eine auf die Querabmessung des Kapselsackes des Empfängerauges abgestimmte Länge hat;
wobei die haptische Ankerplatte (14, 14A, 104) eine Breite hat, die im wesentlichen gleich dem Durchmesser des optischen Teils (12, 102) ist; und
wobei die haptischen Elemente (16, 16A, 16B, 106) bogenförmig gekrümmte, rückstellfähige Elemente (16, 16A, 16B, 106) sind, von denen mindestens eines während des Positionierens der intraokularen Linse (10, 100) in dem Kapselsack nach unten in Richtung auf eine Kante der haptischen Ankerplatte (14, 14A, 104) zusammengedrückt werden kann und wobei mindestens eines der Elemente nach außen weg von der Ankerplatte (14, 14A, 104) für ein alternatives Positionieren der intraokularen Linse in dem Sulcus verschieblich ist, wodurch das mindestens eine Element dazu dient, die intraokulare Linse (10, 100) zentral in dem Auge des Empfängers zu positionieren.

2. Intraokulare Linse (10, 100) nach Anspruch 1, bei welcher die haptischen Bügel-Elemente (16, 16A, 16B, 106) aus Polypropylen gebildet werden.

3. Intraokulare Linse (10, 100) nach Anspruch 1, bei welcher mindestens eines der optischen Teile (12, 102) und die Ankerplatte (14, 14A, 104) aus Hydroxyethylmethylmethacrylat gebildet werden.

4. Intraokulare Linse (10, 100) nach Anspruch 1, bei welcher mindestens eines der optischen Teile (12, 102), die Ankerplatte (14, 14A, 104) und die haptischen Elemente (16, 16A, 16B, 106) aus einem relativ harten, rückstellfähigen Kunststoffmaterial gebildet werden.

5. Intraokulare Linse (10, 100) nach Anspruch 1, bei welcher die haptischen Elemente (16, 16A, 16B, 106) aus Polyimid gebildet werden.

6. Intraokulare Linse (10, 100) nach Anspruch 1, bei welcher mindestens eines der optischen Teile (12, 102), die haptischen Elemente (16, 16A, 16B, 106) und die Ankerplatte (14, 14A, 104) aus Silicon gebildet werden.

7. Intraokulare Linse (10, 100) nach Anspruch 1, bei welcher mindestens eines der optischen Teile (12, 102), die Ankerplatte (14, 14A, 104) und die haptischen Elemente (16, 16A, 16B, 106) aus Silicon gebildet werden.

8. Intraokulare Linse (10, 100) nach einem der vorgenannten Ansprüche, bei welcher die Kanten der haptischen Ankerplatte (14, 14A, 104) an deren entsprechenden Enden darin ausgebildete Rillen aufweisen, um die bogenförmig gekrümmten haptischen Elemente (16, 16A, 16B, 106) aufzunehmen, wenn die haptischen Elemente (16, 16A, 16B, 106) nach unten in die Rillen des Kapselsackes gedrückt werden.

9. Intraokulare Linse (10, 100) nach Anspruch 1, bei welcher der optische Teil (12, 102) aus Zusammenlegbarem Kunststoffmaterial gebildet wird.

10. Intraokulare Linse (10, 100) nach Anspruch 1, bei welcher mindestens eines der optischen Teile (12, 102), die Ankerplatte (14, 14A, 104) und die haptischen Elemente (16, 16A, 16B, 106) aus rückstellfähigem Kunststoffmaterial gebildet werden.

11. Intraokulare Linse (10, 100) nach einem der vorgenannten Ansprüche, welche Linse so konfiguriert ist, daß zwischen jedem der haptischen Elemente (16, 16A, 16B, 106) und der Ankerplatte (14, 14A, 104) ein Zwischenraum gewährt wird, wenn die intraokulare Linse (10, 100) in den Kapselsack eingesetzt ist, um zu ermöglichen, die vorderen und hinteren Kapseln des Auges durch diesen Zwischenraum mit Hilfe von Fibrose zusammenzuschweißen und die intraokulare Linse (10, 100) in dem Auge zu fixieren

12. Intraokulare Linse (10, 100) nach einem der vorgenannten Ansprüche, bei welcher jedes der haptischen Elemente (16, 16A, 16B, 106) an dem einen Ende nur an der Ankerplatte (14, 14A, 104) eingesetzt ist.

13. Intraokulare Linse (10, 100) nach Anspruch 1, bei welcher jedes der bogenförmig gekrümmten, rückstellfähigen haptischen Elemente (16, 16A, 16B, 106) zusammendrückbar ist und an den beiden Enden an der Ankerplatte (14, 14A, 104) angebracht ist.

14. Intraokulare Linse (10, 100) nach Anspruch 12, bei welcher jedes Ende der Ankerplatte (14, 14A, 104) gekerbt ist, um das freie Ende der entsprechenden haptischen Elemente (16, 16A, 16B, 106) aufzunehmen.

15. Intraokulare Linse (10, 100) nach einem der vorgenannten Ansprüche, bei welcher die intraokulare Linse (10, 100) zum Einsetzen durch einen 3 ... 4 mm-Phakoemulsifikationseinschnitt in das Auge des Empfängers zusammendrückbar ist, in den die intraokulare Linse (10, 100) eingesetzt werden soll.

## Revendications

1. Lentille intraoculaire (10,100) pour un oeil d'un receveur, comprenant :
une partie optique sensiblement en forme de disque (12,102) ;
une plaque d'ancrage haptique (14,14A,104) s'étendant radialement vers l'extérieur à partir de ladite partie optique (12,102) dans des directions opposées, et attachée à la dite partie optique ;
deux éléments haptiques (16,16A,16B,106) attachés à ladite plaque d'ancrage (14,14A,104) à ses extrémités opposées, ladite plaque d'ancrage haptique, ladite partie optique et lesdits éléments haptiques étant tous dans un même plan ;
ladite plaque d'ancrage haptique ayant une longueur prévue pour correspondre à la dimension transversale du sac capsulaire de l'oeil du receveur ;
ladite plaque d'ancrage haptique (14,14A,104) ayant une largeur sensiblement égale au diamètre de la dite partie optique (12,102) ; et
lesdits éléments haptiques (16,16A,16B,106) étant des éléments élastiques courbes (16,16A,16B,106) dont au moins un peut être comprimé vers un bord d'extrémité de la plaque d'ancrage haptique (14,14A,104) pendant la mise en place de la lentille intraoculaire (10,100) dans le sac capsulaire et ledit au moins un élément est déplaçable vers l'extérieur de la plaque d'ancrage haptique (14,14A, 104) pour une autre mise en place de la lentille intraoculaire dans le sulcus, de sorte que ledit au moins un élément permet de placer la lentille intraoculaire (10,100) centralement dans l'oeil du receveur.

2. Lentille intraoculaire (10,100) selon la revendication 1, dans lequel lesdits éléments en boucle haptiques (16,16A,16B,106) sont en polypropylène.

3. Lentille intraoculaire (10,100) selon la revendication 1, dans laquelle au moins une de ladite partie optique (12,102) et de ladite plaque d'ancrage (14,14A,104) est en hydroxyéthylméthylméthacrylate.

4. Lentille intraoculaire (10,100) selon la revendication 1, dans laquelle au moins un de ladite partie optique (12,102), de ladite plaque d'ancrage (14, 14A,104) et desdits éléments haptiques (16,16A,16B,106) est en une matière plastique rigide relativement dure.

5. Lentille intraoculaire (10,100) selon la revendication 1, dans laquelle lesdits éléments haptiques (16,16A,16B,106) sont en polyimide.

6. Lentille intraoculaire (10,100) selon la revendication 1, dans laquelle au moins un de ladite partie optique (12,102), desdits éléments haptiques (16, 16A,16B,106) et de ladite plaque d'ancrage (14,14A,104) est en silicone.

7. Lentille intraoculaire (10,100) selon la revendication 1, dans laquelle au moins un de ladite partie optique (12,102), de ladite plaque d'ancrage (14, 14A,104) et desdits éléments haptiques (16,16A,16B,106) est en une matière plastique semi-rigide.

8. Lentille intraoculaire (10,100) selon une quelconque des revendications précédentes, dans laquelle les bords de ladite plaque d'ancrage haptique (14,14A, 104),à ses extrémités respectives, comportent des gorges pour la réception des éléments haptiques courbes (16, 16A,16B,106) lorsque les éléments haptiques (16,16A,16B, 106) sont comprimés dans les gorges par le sac capsulaire.

9. Lentille intraoculaire (10,100) selon la revendication 1, dans laquelle ladite partie optique (12, 102) est en matière plastique pliable.

10. Lentille intraoculaire (10,100) selon la revendication 1, dans laquelle au moins un de ladite partie optique (12,102), de ladite plaque d'ancrage (14, 14A,104) et desdits éléments haptiques (16,16A,16B,106) est en une matière plastique élastique.

11. Lentille intraoculaire (10,100) selon une quelconque des revendications précédentes, configurée de sorte qu'un espace est prévu entre chacun des éléments haptiques (16,16A,16B,106) et la plaque d'ancrage (14,14A,104) lorsque la lentille intraoculaire (10,100) est insérée dans le sac capsulaire, pour permettre aux capsules antérieure et postérieure de l'oeil de fusionner mutuellement à travers ledit espace par fibrose et de fixer solidement la lentille intraoculaire (10,100) dans l'oeil.

12. Lentille intraoculaire (10,100) selon une quelconque des revendications précédentes, dans laquelle chacun desdits éléments haptiques (16,16A,16B,106) est attaché à une extrémité seulement à ladite plaque d'ancrage (14,14A,104).

13. Lentille intraoculaire (10,100) selon la revendication 1, dans laquelle chacun desdits éléments haptiques élastiques courbes (16,16A,16B,106) est compressible et attaché aux deux extrémités à ladite plaque d'ancrage (14,14A,104).

14. Lentille intraoculaire (10,100) selon la revendication 12, dans laquelle chaque extrémité de ladite plaque d'ancrage (14,14A,104) présente une encoche pour recevoir l'extrémité libre des éléments haptiques correspondants (16,16A,16B,106).

15. Lentille intraoculaire (10,100) selon une quelconque des revendications précédentes, dans laquelle la lentille intraoculaire (10,100) est compressible pour insertion à travers une incision de phacoémulsification de 3 à 4 mm dans l'oeil du receveur dans lequel on veut insérer la lentille intraoculaire (10,100).
